# EUROPEAN PATENT SPECIFICATION

(11) **EP 2 468 760 B1**
(45) Date of publication and mention of the grant of the patent: **01.10.2014**
(21) Application number: 10809736.1
(22) Date of filing: 19.08.2010
(51) Int. Cl.: C07F 9/6515, A61K 47/24, A61K 51/04, C07B 59/00, C07F 5/00, C07F 9/6524, C07F 13/00

(54) **BISPHOSPHONIC ACID DERIVATIVE AND COMPOUND THEREOF LABELED WITH RADIOACTIVE METAL NUCLIDE**
BISPHOSPHONSÄUREDERIVAT UND MIT EINEM RADIOAKTIVEN METALLNUKLID MARKIERTE VERBINDUNG DARAUS
DÉRIVÉ D ACIDE BISPHOSPHONIQUE ET COMPOSÉ DE CELUI-CI MARQUÉ AVEC UN NUCLÉIDE MÉTALLIQUE RADIOACTIF

(30) Priority: 20.08.2009 JP 2009190714
(43) Date of publication of application: 27.06.2012
(73) Proprietor: Fujifilm RI Pharma Co., Ltd., Tokyo 104-0031 (JP)
(72) Inventor: DOZONO, Hiroyuki, Sammu-shi Chiba 289-1592 (JP)
(74) Representative: Blodig, Wolfgang
(86) International application number: PCT/JP2010/005111
(87) International publication number: WO 2011/021390

(56) References cited:
- WO-A1-2005/053674
- JP-A- H02 501 069
- JP-A- H05 504 127
- JP-A- H06 502 645
- TOMÁS VITHA ET AL: "Lanthanide(III) Complexes of Bis(phosphonate) Monoamide Analogues of DOTA: Bone-Seeking Agents for Imaging and Therapy", JOURNAL OF MEDICINAL CHEMISTRY, vol. 51, no. 3, 1 February 2008 (2008-02-01), pages 677-683, XP055045560, ISSN: 0022-2623, DOI: 10.1021/jm7012776
- VELIKYAN I ET AL: "Convenient preparation of <68>Ga-based PET-radiopharmaceuticals at room temperature", BIOCONJUGATE CHEMISTRY, ACS, WASHINGTON, DC, US, vol. 19, no. 2, 1 February 2008 (2008-02-01), pages 569-573, XP002546370, ISSN: 1043-1802, DOI: 10.1021/BC700341X [retrieved on 2008-01-19]

## Description

### Technical Field

The present invention relates to a bisphosphonic acid derivative having a bone-seeking bisphosphonic acid compound linked to a cyclic chelate via a spacer, a bisphosphonic acid derivative labeled with a radioactive metal nuclide in which a radioactive metal nuclide coordinates to the derivative, and a radioactive bone diagnostic agent or a radioactive bone therapeutic agent having a bisphosphonic acid derivative labeled with a radioactive metal nuclide as an active ingredient.

### Background Art

Recently, bone scintigraphy using radiopharmaceuticals has become an important examination for the diagnosis of early-stage bone disease. As an imaging agent for bone scintigraphy, technetium 99m (99mTc)-labeled organic diphosphonic acid compounds such as methanediphosphonic acid (MDP) and hydroxymethanediphosphonic acid (HMDP) are widely employed. In order to shorten the time from after administration of pharmaceuticals to imaging and obtain high-contrast scintigraphic images, these radioactive bone diagnostic agents are required not only bone-seeking but also rapid urinary excretion and clearance from blood and tissues, and the like.

In general, when an imaging agent used for bone scintigraphy has slow clearance from blood or soft tissues or slow urinary excretion, the time from after administration of pharmaceuticals to imaging is prolonged since time is needed to reduce the background.

Presently used 99mTc-labeled organic diphosphonic acid compounds form a polymer structure through formation of polynuclear complexes by using 99mTc, which is a radioactive metal, and organic diphosphonic acid compound. Thus, it is highly likely that this polymer structure formation affects clearance from blood and soft tissues.

Meanwhile, bone scintigraphy using fluorine 18 (18F), which is a positron (PET) nuclide, has also been reported as a diagnostic agent for bone metastasis since 1960s (Non Patent Document 1).

Sodium fluoride 18 (18F-Na) was approved as a pharmaceutical product by Food and Drug Administration (FDA); however, because it was imaged by a normal gamma camera, the image quality was worse than 99mTc, and moreover, acquisition of 18F-Na was difficult. Due to the foregoing, 18F-Na was immediately replaced by 99mTc bone preparations once they were out. At present, 99mTc preparations are used as the standard diagnostic imaging agent for the diagnosis of bone metastasis. However, as specialized cameras for PET have become popular since 1990s, the excellent features of 18F-Na/PET have once again come into the spotlight such that PET images taken with 18F-Na have better contrast and spatial resolution than existing images taken with the 99mTc preparations and while the 99mTc preparations require a waiting time of three hours after administration, 18F-Na enables imaging one hour after administration.

However, there are problems that a cyclotron is needed to produce 18F, its facilities are limited to those dedicated and controlled and examination facilities need to be established near the cyclotron.

However, gallium 68 (68Ga), which is also a PET nuclide as 18F is, is obtainable from a generator using 68Ge/68Ga, and is easily obtainable at the site by simple milking operation using the generator, and thus is advantageous in not requiring a cyclotron, which is needed for the production of 18F.

As the radioactive bone diagnostic agent using 68Ga, 68Ga-ethylene diamine tetramethylene phosphonate (EDTMP), which is a polyphosphoric acid compound, was reported first (Non Patent Documents 2 and 3). Also, an attempt to improve clearance performance, i.e., early clearance after administration, using a compound having both a bisphosphonic acid compound and a chelate moiety which forms a complex with radioactive metal, application of such a compound to pain relief therapy for bone metastasis, and the like are reported (Patent Documents 1 and 2 and Non Patent Documents 4 to 6). However, sufficient effects have not necessarily been achieved, and none of them has ever been put to practical use.

Further, compared to existing radioactive metal, 68Ga has a short half-life of 68 minutes, and thus shortening of time from administration of drugs to imaging is a crucial problem.

Also, studies on a compound labeled with 68Ga using 1,4,7-triazacyclononane-N,N',N"-triacetic acid (NOTA) as a chelating agent are reported (Patent Document 3 and Non Patent Documents 7 and 8). However, the compounds used in these studies are mostly peptide compounds, and further, none of them reports bone-seeking compounds.

### Prior Art Documents

### Patent Document

Patent Document 1: JP-A-2001-114792
Patent Document 2: JP-A-2005-263690
Patent Document 3: JP-A-2005-514444

### Non Patent Document

Non Patent Document 1: J. Nucl. Med., 3, 332 (1962)
Non Patent Document 2: J. Nucl. Med., 17, 1003 (1976)
Non Patent Document 3: Nucl. Med. Biol., 34, 391 (2007)
Non Patent Document 4: J. Nucl. Med., 47, 2042 (2006)
Non Patent Document 5: Nucl. Med. Biol., 36, 129 (2009)
Non Patent Document 6: J. Med. Chem., 51, 677 (2008)
Non Patent Document 7: Bioconjugate Chem., 19, 569 (2008)
Non Patent Document 8: J. Nucl. Med., 49, 830 (2008)

### Summary of Invention

### Problem to be Solved by the Invention

In view of the foregoing circumstances, a problem of the present invention is to provide a radioactive bone diagnostic agent which gives a high ratio of radioactivity accumulation in bone to that in blood from an early stage after administration of the agent and allows capturing an image in a short time after administration.

### Means for Solving the Problem

The present inventors conducted various studies to solve the aforementioned problem. As a result, they have found that a bisphosphonic acid derivative, which is obtained by linking 4-amino-l-hydroxybutylidene-1,1-bisphosphonate (generic name: alendronate), a bone-seeking bisphosphonic acid compound, to a cyclic chelate, NOTA, via a spacer, is used as a labeling precursor and is labeled with a radioactive metal nuclide, 68Ga, and a radioactive bone diagnostic agent containing, as an active ingredient, the bisphosphonic acid derivative labeled with a radioactive metal nuclide gives a high ratio of radioactivity accumulation in bone to that in blood from an early stage after administration of the agent and allows capturing a clear image of bone in a short time after administration. They have also found that a bisphosphonic acid derivative labeled with a radioactive metal nuclide obtained by labeling the aforementioned labeling precursor labeled with a therapeutic radioactive nuclide such as 64Cu, is useful for the treatment of a bone metastatic focus of tumor.

That is, the present invention provides a bisphosphonic acid derivative represented by the chemical formula (II): 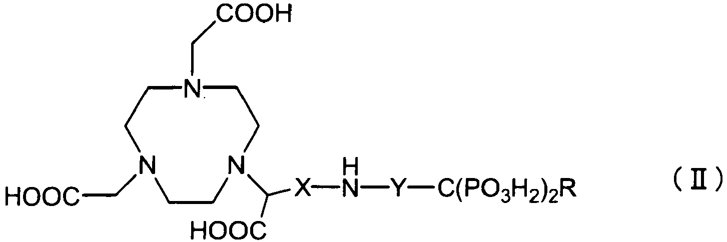 or a salt thereof, (wherein X represents -(CH₂)ₘCO-, Y represents -(CH₂)ₙ-, R represents H, OH, or a halogen atom, m and n are independent of each other and m represents an integer of 1 to 3, and n represents an integer of 0 to 4).

The present invention also provides a bisphosphonic acid derivative represented by the chemical formula (I): 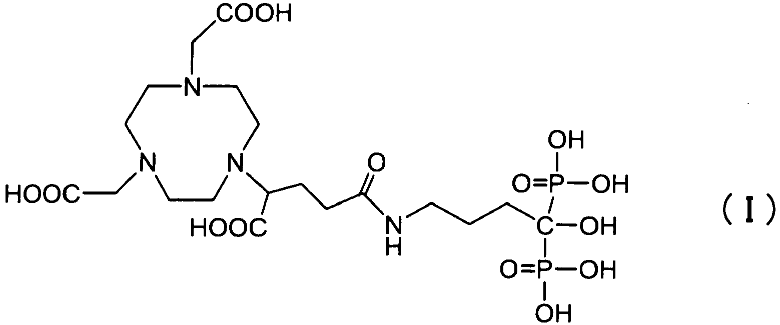 or a salt thereof.

The present invention also provides a bisphosphonic acid derivative labeled with a radioactive metal nuclide or a salt thereof, wherein a radioactive metal nuclide coordinates to the bisphosphonic acid derivative represented by the aforementioned formula (II) or (I), or a salt thereof.

The present invention also provides a radioactive bone diagnostic agent containing, as an active ingredient, the aforementioned bisphosphonic acid derivative labeled with a radioactive metal nuclide or a salt thereof, wherein the radioactive metal nuclide is selected from 62Cu, 64Cu, 67Ga, 68Ga, and 111In.

The present invention also provides a radioactive bone therapeutic agent containing, as an active ingredient, the aforementioned bisphosphonic acid derivative labeled with a radioactive metal nuclide or a salt thereof, wherein the radioactive metal nuclide is selected from 64Cu, 67Cu, 177Lu, 153Sm, and 90Y.

The present invention also provides a kit for preparation of a diagnostic or therapeutic radioactive agent, which comprise the bisphosphonic acid derivative represented by the aforementioned formula (II) or (I) or a salt thereof and is prepared by addition of a radioactive metal nuclide.

The present invention also provides the aforementioned bisphosphonic acid derivative labeled with a radioactive metal nuclide or a salt thereof, for use in radioactive bone diagnosis, wherein the radioactive metal nuclide is selected from 62Cu, 64Cu, 67Ga, 68Ga, and 111In.

The present invention also provides the aforementioned bisphosphonic acid derivative labeled with a radioactive metal nuclide or a salt thereof, for use in radioactive bone therapy, wherein the radioactive metal nuclide is selected from 64Cu, 67Cu, 177Lu, 153Sm, and 90Y.

The present invention also provides a method of radioactive bone diagnosis, characterized in that the method comprises administrating an effective amount of the aforementioned bisphosphonic acid derivative labeled with a radioactive metal nuclide or a salt thereof, wherein the radioactive metal nuclide is selected from 62Cu, 64Cu, 67Ga, 68Ga, and 111In, to a subject in need thereof.

Also described is a method of radioactive bone therapy, characterized in that the method comprises administrating an effective amount of the aforementioned bisphosphonic acid derivative labeled with a radioactive metal nuclide or a salt thereof, wherein the radioactive metal nuclide is selected from 64Cu, 67Cu, 177Lu, 153Sm, and 90Y, to a subject in need thereof.

### Advantageous Effects of Invention

Compared to conventional radioactive bone diagnostic agents (for example, 99mTc-MDP), the radioactive bone diagnostic agent of the present invention shows equivalent accumulation in bone, while having faster clearance from blood. Thus, a high ratio of radioactivity accumulation in bone/blood is obtainable from an early stage after administration, enabling shortening of the time up to imaging. Further, taking advantage of the characteristics of a PET nuclide, the radioactive bone diagnostic agent of the present invention allows easy tomographic imaging of the whole body, holding promise for improving the bone disease diagnostic capability.

### Brief Description of Drawings

[Figure 1] Figure 1 shows the in vivo distribution of 68Ga-NOTA-alendronate, 99mTc-MDP, and 18F-Na in a rat.
[Figure 2] Figure 2 shows the in vivo distribution of 68Ga-NOTA-alendronate, 99mTc-MDP, and 18F-Na in a rat.
[Figure 3] Figure 3 shows the in vivo distribution of 68Ga-NOTA-alendronate, 99mTc-MDP, and 18F-Na in a rat.
[Figure 4] Figure 4 shows images of 99mTc-MDP in a rat.
[Figure 5] Figure 5 shows images of 68Ga-NOTA-alendronate in a rat.
[Figure 6] Figure 6 shows images of 68Ga-NOTA-alendronate in a model mouse of local bone metastasis.

### Modes for Carrying Out the Invention

The bisphosphonic acid derivative of the present invention represented by the chemical formulas (II) and (I) or a salt thereof is useful as a precursor for coordinate bond to a radioactive metal nuclide. In the formula (II), X represents -(CH₂)ₘCO-, wherein m is an integer of 1 to 3. More preferably, X represents - (CH₂)₂CO-.

Y represents -(CH₂)ₙ-, wherein n is an integer of 0 to 4. More preferably, Y represents - (CH₂)₂- or - -(CH₂)₃-, and particularly preferably, Y represents -(CH₂)₃-.

Accordingly, among the bisphosphonic acid derivatives of the formula (II), the compound of the formula (I) is particularly preferred.

Examples of the salt of the compound of the formula (II) include alkali metal salt such as sodium salt and potassium salt and alkaline earth metal salt such as calcium salt.

The compound of the formula (I) can be obtained by, for example, the following process. Firstly, an alendronate derivative having hydroxyl groups protected by an ethyl group and a tert-butyldimethylsilyl group (a TBS group) is allowed to undergo condensation reaction with a NOTA derivative having a carboxyl group protected by a tert-butyl group (a t-Bu group). Subsequently, each protecting group is removed, whereby NOTA-alendronate, which is the bisphosphonic acid derivative of the formula (I), can be produced.

It is noted that the use of raw materials with different lengths of methylene chain in the synthesis of the alendronate derivative can produce bisphosphonic acid derivatives with different lengths of the methylene chain between the P-C-P skeleton and the amino group. Also, it is noted that similarly, the use of raw materials with different lengths of methylene chain in the synthesis of NOTA derivative can produce bisphosphonic acid derivatives with different lengths of spacer.

The compound of the formula (II) or (I) is dissolved in a sodium acetate solution, a sodium acetate buffer, and the like and 68GaCl₃ eluted from a 68Ge/68Ga generator is added thereto to carry out a reaction, which gives a bisphosphonic acid derivative labeled with a radioactive metal nuclide. Bisphosphonic acid derivatives labeled with a radioactive metal nuclide which various radioactive nuclides are coordinated to or salts thereof are obtained by using other radioactive nuclides in place of 68Ga.

Examples of the radioactive metal nuclide used include 62Cu, 64Cu, 67Cu, 67Ga, 68Ga, 177Lu, 153Sm, 90Y, and 111In. Among them, the use of 62Cu, 64Cu, 67Ga, 68Ga, and 1111n can yield a bisphosphonic acid derivative labeled with a radioactive metal nuclide or a salt thereof useful as a radioactive bone diagnostic agent. Meanwhile, the use of 64Cu, 67Cu, 177Lu, 153Sm, and 90Y can yield a bisphosphonic acid derivative labeled with a radioactive metal nuclide or a salt thereof useful as a radioactive bone therapeutic agent.

According to the present invention, a bisphosphonic acid derivative labeled with a radioactive metal nuclide having a radiochemical purity of 95% or higher is obtainable. Thus, without any special purification operation, the reaction solution can be directly used as the radioactive bone diagnostic agent or the radioactive bone therapeutic agent.

The radioactive bone diagnostic agent or radioactive bone therapeutic agent of the present invention can contain, besides the active ingredient, pH adjusters, surfactants, stabilizers, buffering agents, excipients, and the like within a clinically acceptable range.

The bisphosphonic acid derivative of the present invention is provided as an aqueous solution preparation or a freeze dried preparation, and a kit preparation for the preparation of the radioactive bone diagnostic agent can be given by providing the aqueous solution preparation or the freeze dried preparation together with buffering agents, stabilizers, and the like. Particularly preferably, the kit preparation for the preparation of the radioactive bone diagnostic agent including the bisphosphonic acid derivative of the present invention is provided as a freeze dried preparation since it can be prepared immediately before use simply by adding a 68GaCl₃ solution, which is easily obtained from a 68Ge/68Ga generator. At the time of use, the freeze dried preparation is dissolved to carry out labeling, and the solution thus obtained is used for administration.

The radioactive bone diagnostic agent or radioactive bone therapeutic agent of the present invention can be used in a similar manner to conventional radioactive bone diagnostic agents or radioactive bone therapeutic agents. For example, a liquid agent is used for mammals including humans by administration. Similarly to conventional radioactive bone diagnostic agents or radioactive bone therapeutic agents, the dose is 1.85 to 12.3 MBq/kg, preferably 3.1 to 6.2 MBq/kg. The dose is appropriately increased or decreased in consideration of the age, body weight, and symptoms of the patient, administration method, and combined use of other drugs.

The radioactive bone therapeutic agent of the present invention is useful for the treatment of a bone metastatic focus of tumor diagnosed by the aforementioned radioactive bone diagnostic agent of the present invention.

Hereinbelow, the present invention will be described in detail with reference to Examples. However, the present invention is not limited to these Examples.

### Examples

### Example 1

### Synthesis of 4-amino-1-{[(tert-butyl)-1,1-dimethylsilyl]oxy}-1,1-di(diethoxyphosphoryl)-butane (4)

The compound (4) was prepared by the steps 1 to 4 as shown below.

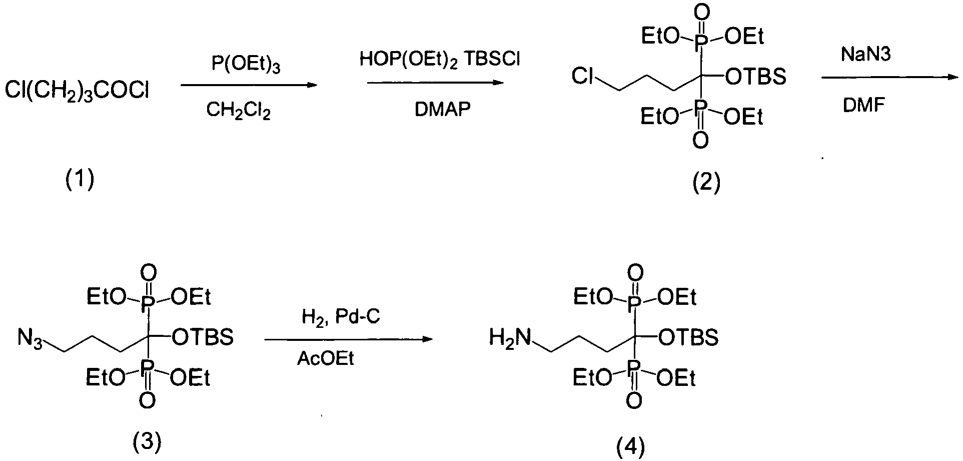

### Synthesis of 1-{[(tert-butyl)-1,1-dimethylsilyl]oxy}-4-chloro-1,1-di(diethoxyphosphoryl)-butane (2)

To triethyl phosphite (3.3 g), 4-chlorobutyl chloride (1) (2.82 g) was added dropwise, followed by stirring. To the resulting reaction solution, dichloromethane (100 mL) was added, to which diethyl phosphite (3.0 g) was further added dropwise, followed by stirring. To the resulting reaction solution, N,N-dimethylaminopyridine (2.44 g) and tert-butyldimethylsilyl chloride (3.32 g) were added, followed by stirring. After distilling off the solvent, the remaining product was dissolved in ethyl acetate and washed with an aqueous solution of hydrochloric acid. The resulting solution was dried and the solvent was distilled off, and the residue thus obtained was purified by silica gel column chromatography to give the title compound (2.0 g).
¹H-NMR (solvent: deuterated chloroform, resonance frequency: 400MHz) δ: 0.21 (6H, s), 0.91 (9H, s), 1.35 (12H, t, J=7.1Hz), 2.15-2.22 (4H, m), 3.54-3.57 (2H, m), 4.17-4.26 (8H, m).

### Synthesis of 4-azide-1-{[(tert-butyl)-1,1-dimethylsilyl]oxy}-1,1-di(diethoxyphosphoryl)-butane (3)

To a dimethylformamide solution (50 mL) containing 1-{[(tert-butyl)-1,1-dimethylsilyl]oxy}-4-chloro-1,1-di(diethoxyphosphoryl)-butane (2) (3.7g), sodium azide (963 mg) was added, followed by stirring. To the resulting reaction solution, ethyl acetate was added, followed by washing with water. After that, the resulting solution was dried, the solvent was distilled off, and the residue thus obtained was purified by silica gel column chromatography to give the title compound (1.4 g).
¹H-NMR (solvent: deuterated chloroform, resonance frequency: 400MHz) δ: 0.20 (6H, s), 0.91 (9H, s), 1.35 (12H, t, J=7.1Hz), 1.96-2.13 (4H, m), 3.27 (2H, t, J=6.6Hz), 4.19-4.26 (8H, m).

### Synthesis of 4-amino-1-{[(tert-butyl)-1,1-dimethylsilyl]oxy}-1,1-di(diethoxyphosphoryl)-butane (4)

To 4-azide-1-{[(tert-butyl)-1,1-dimethylsilyl]oxy}-1,1-di(diethoxyphosphoryl)-butane (3) (1.4 g) dissolved in ethyl acetate (50 ml), 10% palladium carbon (300 mg) was added, followed by stirring under a hydrogen stream. The resulting reaction solution was filtered and concentrated, and the residue thus obtained was purified by silica gel column chromatography to give the title compound (780 mg).
¹H-NMR (solvent: deuterated chloroform, resonance frequency: 400MHz) δ: 0.20 (6H, s), 0.91 (9H, s), 1.34 (12H, t, J=7.1Hz), 1.78-1.82 (2H, m), 1.99-2.08 (2H, m), 2.67 (2H, t, J=7.1Hz), 4.18-4.25 (8H, m).

### Example 2

### Synthesis of 2-[4,7-di(carboxymethyl)-1,4,7-triazonan-1-yl]-5-[(4-hydroxy-4,4-diphosphonobutyl)amino]-5-oxopentanoic acid (11: NOTA-alendronate)

The compound (11) was prepared by the steps 5 to 11 as shown below.

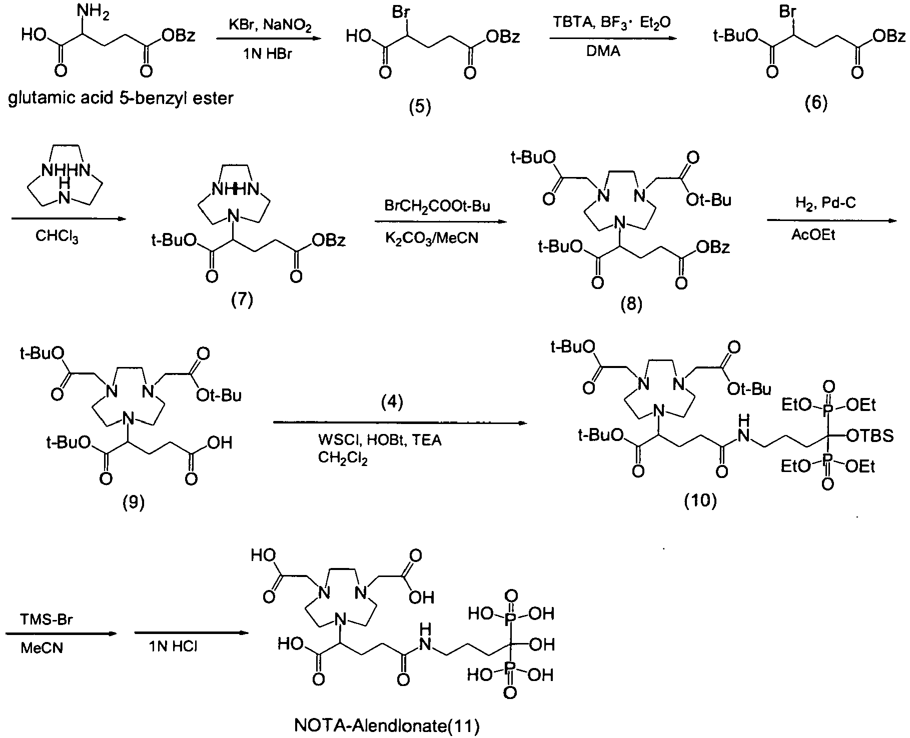

### Synthesis of 5-(benzyloxy)-2-bromo-5-oxopentanoic acid (5)

Glutamic acid-5-benzyl ester (7.0 g) and potassium bromide (10.5 g) were dissolved in an aqueous solution of hydrobromic acid (60 mL), followed by stirring. To the resulting reaction solution, sodium nitrite (4.1 g) was added, followed by stirring. To the resulting reaction solution, concentrated sulfuric acid (3 mL) was added, followed by extraction with ethyl acetate. The resulting ethyl acetate layer was washed with brine and then dried, and the solvent was distilled off. The residue thus obtained was purified by silica gel column chromatography to give the title compound (5.3 g).
¹H-NMR (solvent: deuterated chloroform, resonance frequency: 400MHz) δ: 2.28-2.44 (2H, m), 2.58-2.62 (2H, m), 4.39-4.43 (1H, m), 5.14 (2H, s), 7.33-7.37 (5H, m).

### Synthesis of 5-benzyl-1-(tert-butyl)-2-bromopentanedioate (6)

To 5-(benzyloxy)-2-bromo-5-oxopentanoic acid (5) (5.3 g) dissolved in chloroform, tert-butyl trichloroacetimidate (6.95 g) dissolved in cyclohexane (25 mL) was added dropwise. Upon completion of the reaction, dimethylacetamide (4 mL) was added dropwise, and subsequently, boron trifluoride-diethyl ether (0.4 mL) was added dropwise, followed by stirring. After distilling off the solvent, hexane was added to the remaining dimethylacetamide layer to conduct extraction. The resulting organic layer was dried, the solvent was distilled off, and the residue thus obtained was purified by silica gel column chromatography to give the title compound (4.6 g).
¹H-NMR (solvent: deuterated chloroform, resonance frequency: 400MHz) δ: 1.47 (9H, s), 2.25-2.37 (2H, m), 2.53-2.58 (2H, m), 4.22-4.26 (1H, m), 5.13 (2H, s), 7.32-7.36 (5H, m).

### Synthesis of 5-benzyl-1-(tert-butyl)-2-(1,4,7-triazonan-1-yl)pentanodioate (7)

To 1,4,7-tetraazacyclononane (700 mg) dissolved in chloroform, 5-benzyl-1-(tert-butyl)-2-bromopentanedioate (6) (643 mg) dissolved in chloroform was added dropwise. After stirring, the solvent was distilled off, and the residue thus obtained was purified by silica gel column chromatography to give the title compound (540 mg). ¹H-NMR (solvent: deuterated chloroform, resonance frequency: 400MHz) δ: 1.46 (9H, s), 1.91-2.09 (2H, m), 2.50-2.58 (2H, m), 2.65-2.84 (12H, m), 3.19-3.23 (1H, m), 5. 14 (2H, s), 7.25-7.36 (5H, m).

### Synthesis of 5-benzyl-1-(tert-butyl)-2-{4,7-di[2-tert-butoxy)-2-oxoethyl]-1,4,7-triazonan-1-yl}pentanedioate (8)

To 5-benzyl-1-(tert-butyl)-2-(1,4,7-triazonan-1-yl)pentanodioate (7) (840 mg) dissolved in acetonitrile, tert-butyl bromoacetate (579 µL) was added. Upon completion of the reaction, potassium carbonate (1.63 g) was added, followed by stirring. The resulting reaction solution was filtered through Celite and eluted with acetonitrile. The filtrate was concentrated and the residue thus obtained was purified by silica gel column chromatography to give the title compound (1.2 g). ¹H-NMR (solvent: deuterated chloroform, resonance frequency: 400MHz) δ: 1.48 (27H, s), 1.87-2.05 (2H, m), 2.47-2.63 (2H, m), 2.68-2.94 (12H, m), 3.16-3.19 (1H, m), 3.27 (4H, s), 5.12 (2H, m), 7.26-7.36 (5H, m).

### Synthesis of 5-(tert-butoxy)-4-{4,7-di[2-(tert-butoxy)-2-oxoethyl]-1,4,7-triazonan-1-yl}-5-oxopentanoic acid (9)

To 5-benzyl-1-(tert-butyl)-2-{4,7-di[2-tert-butoxy)-2-oxoethyl]-1,4,7-triazonan-1-yl}pentanedioate (8) (1.2 g) dissolved in isopropanol, water and 10% palladium carbon (500 mg) were added, followed by stirring under a hydrogen stream. The resulting reaction solution was filtered through Celite, the filtrate was concentrated, and the residue thus obtained was purified by silica gel column chromatography to give the title compound (980 mg). ¹H-NMR (solvent: deuterated chloroform, resonance frequency: 400MHz) δ: 1.45 (18H, s), 1.48 (9H, s), 1.94-2.00 (2H, m), 2.48-2.56 (2H, m), 2.65-3.12 (12H, m), 3.33-3.35 (1H, m), 3.41 (4H, s).
ESI-MS (posi) m/z: 544 (M+H)⁺.

### Synthesis of tert-butyl-5-{[4-{[1-(tert-butyl)-1,1-dimethylsilyl]oxy}-4,4-di(diethoxyphosphoryl)butyl]amino}-2-{4,7-di[2-(tert-butoxy)-2-oxoethyl]-1,4,7-triazonan-1-yl}-5-oxopentanoate (10)

To dichloromethane, 4-amino-1-{[(tert-butyl)-1,1-dimethylsilyl]oxy}-1,1-di(diethoxyphosphoryl)-butane (4) (130 mg) and 5-(tert-butoxy)-4-{4,7-di[2-(tert-butoxy)-2-oxoethyl]-1,4,7-triazonan-1-yl}-5-oxopentanoic acid (9) (167 mg) were dissolved, to which 1-ethyl-3-(3-dimethylaminopropyl)carbodiimide (58 mg) and 1-hydroxybenzotriazole (46 mg) were added, followed by stirring at room temperature for 24 hours. After distilling off the solvent, the resulting reaction solution was dissolved in ethyl acetate and then washed with water. The resulting solution was then dried and the solvent was distilled off. The crude product thus obtained was purified by silica gel column chromatography to give the title compound (220 mg).
ESI-MS (posi) m/z: 1002 (M+H)⁺.

### Synthesis of 2-[4,7-di(carboxymethyl)-1,4,7-triazonan-1-yl]-5-[(4-hydroxy-4,4-diphosphonobutyl)amino]-5-oxopentanoic acid (11)

To tert-butyl 5-{[4-{[1-(tert-butyl)-1,1-dimethylsilyl]oxy}-4,4-di(diethoxyphosphoryl)butyl]amino}-2-{4,7-di[2-(tert-butoxy)-2-oxoethyl]-1,4,7-triazonan-1-yl}-5-oxopentanoate (10) (360 mg) dissolved in acetonitrile, bromotrimethylsilane (2 mL) was added, followed by stirring at room temperature for 24 hours. Subsequently, water was added, followed by stirring at room temperature for three hours, and the solvent was distilled off. Water and acetonitrile were sequentially added to the residue thus obtained, and the precipitate was collected by filtration. To the solid thus obtained, a 1N aqueous solution of hydrochloric acid was added, followed by stirring at room temperature for 24 hours. The solvent was distilled off and water and acetonitrile were sequentially added to the residue thus obtained, and the precipitate was collected by filtration to give the title compound (200 mg).
¹H-NMR (solvent: deuterated water, resonance frequency: 400MHz) δ: 1.81-2.16 (6H, m), 2.45 (2H, t, J=7.3Hz), 3.11-3.28 (14H, m), 3.61-3.64 (1H, m), 3.89 (4H, s). ESI-MS (nega) m/z: 302 (M-2H)²⁻, 605 (M-H)⁻.

### Example 3

### Synthesis of 68Ga-NOTA-alendronate

Into a reaction container, 67 µL of sodium acetate buffer (pH 5.5) was added, to which 213 µL of a 68GaCl₃ solution was then added. Subsequently, 20 µL of NOTA-alendronate dissolved in sodium acetate buffer (pH 5.5) was added, and allowed to react at 100°C for 10 minutes. The resulting product was then left to stand at room temperature. The radiochemical purity was found to be 95% or higher as analyzed by TLC and HPLC.

### Analytical instruments and reagents

NMR apparatus: JNM-AL400 model (manufactured by JEOL Ltd.)
ESI-MS apparatus: microTOF (manufactured by Bruker Daltonics)
68Ge/68Ga generator: IGG-100 (trade name, manufactured by Eckert & Ziegler AG)
Eluent: 0.1 N aqueous solution of hydrochloric acid
TLC analysis conditions:
TLC plate: PEI cellulose plate (trade name, manufactured by Merck)
Developing phase: acetone / 2 N aqueous solution of hydrochloric acid = 50 / 50
Detector: radiochromanizer (model: GITA-star, manufactured by raytest)
HPLC analysis conditions:
Column: COSMOSIL HILIC (trade name, manufactured by Nacalai tesque, Inc., size: 4.6 × 150 mm)
Mobile phase: 10 mM phosphoric acid buffer (pH 7) containing 5 mM ethylenediaminetetraacetic acid (EDTA) / acetonitrile = 45 / 55
Flow rate: 1.0 mL/min

### Example 4

### Production of a kit preparation for synthesis of 68Ga-NOTA-alendronate

Into an aqueous solution of disodium phosphate (0.25 M), NOTA-alendronate was dissolved to prepare a 0.376 mM aqueous solution. The resulting solution was dispensed into vials in 3 mL aliquots and then freeze dried, whereby a kit preparation for the synthesis was produced. The reaction with 68Ga was carried out by adding 6 mL of a 68GaCl3 solution to the vial, allowing a reaction to proceed at 150°C for 10 minutes, and then leaving the resulting product to stand at room temperature. The radiochemical purity was found to be 95% or higher as analyzed by TLC and HPLC.

### Example 5

### Rat in vivo distribution experiment

To groups, each containing three Wistar male rats, 0.2 ml of each of 68Ga-NOTA-alendronate, 99mTc-MDP (product name: Techne MDP injection, manufactured by FUJIFILM RI Pharma Co., Ltd., 740 MBq/2 mL), and 18F-Na (provided by Yokohama City University, 90 MBq/mL), each diluted to 5.25 to 12.3 MBq/mL, was administered into the rat tail vein under anesthesia. Subsequently, 15, 30, 60, 120, and 240 minutes after administration, the rats were sacrificed, organs were excised, and the weight and the radioactivity of each organ were measured. The measurement values were calculated as %I.D./g tissue weight, which is the radioactivity in each tissue divided by the tissue weight, as the ratio with respect to the dose.

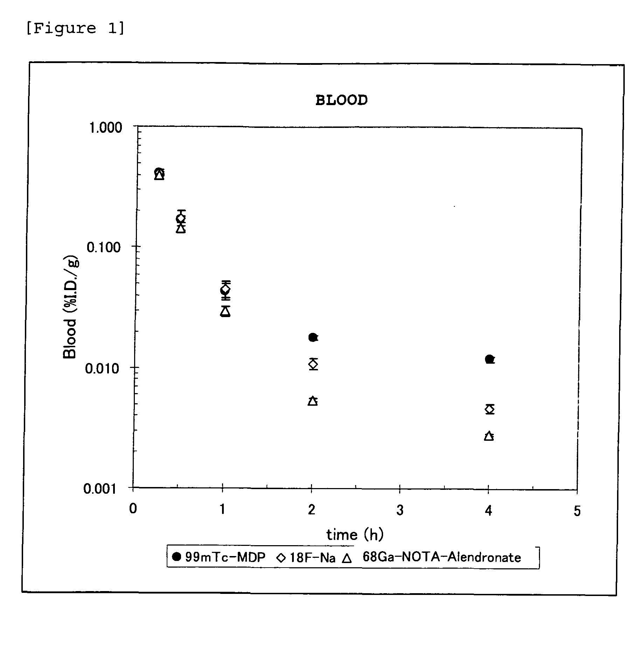

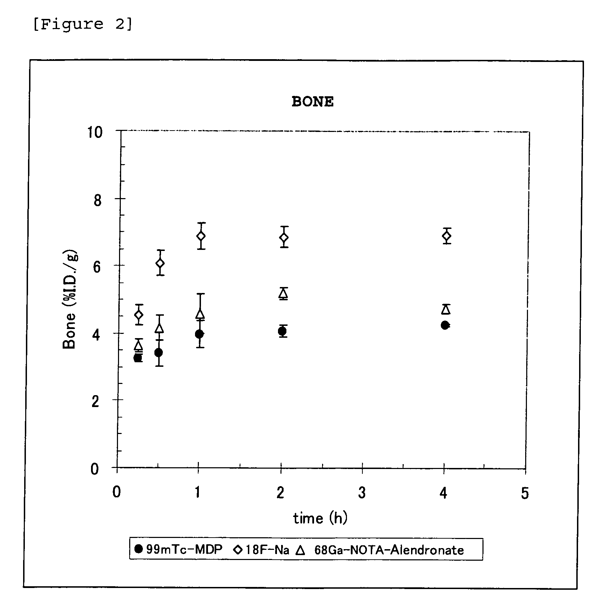

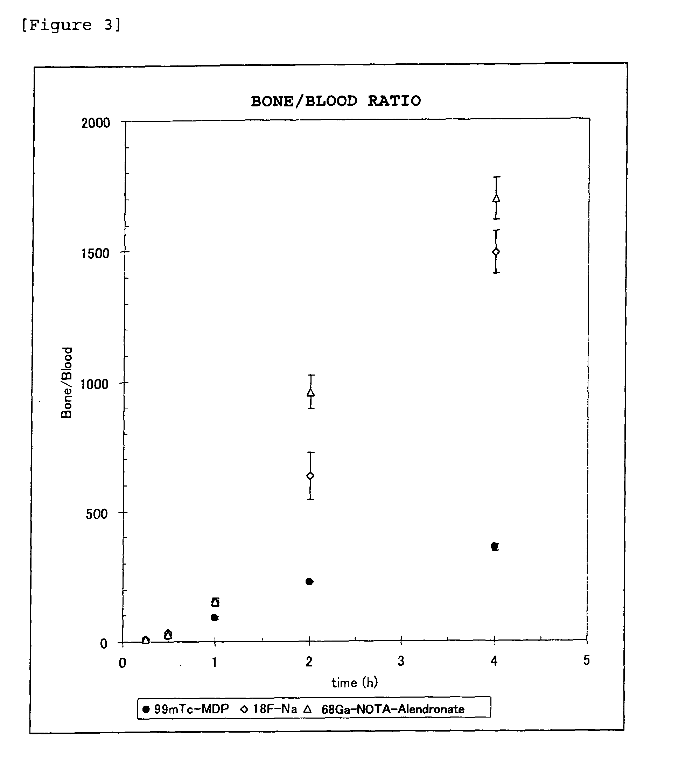

The results thus obtained are shown in Tables 1 to 3 and Figures 1 to 3.

**[Table 1]**

| 99mTc-MDP | | | | | |
|---|---|---|---|---|---|
| | Time after administration | | | | |
| %I. D. /g | 15 min | 30 min | 60 min | 120 min | 240 min |
| Blood | 0.415 | 0.171 | 0.044 | 0.018 | 0.012 |
| | 0.02 | 0.01 | 0.01 | 0.00 | 0.00 |
| Liver | 0.086 | 0.048 | 0.029 | 0.022 | 0.019 |
| | 0.00 | 0.00 | 0.00 | 0.00 | 0.00 |
| Spleen | 0.107 | 0.056 | 0.027 | 0.019 | 0.019 |
| | 0.01 | 0.00 | 0.01 | 0.00 | 0.00 |
| Small intestine | 0.134 | 0.093 | 0.081 | 0.299 | 0.056 |
| | 0.01 | 0.01 | 0.01 | 0.06 | 0.02 |
| Large intestine | 0.121 | 0.062 | 0.020 | 0.053 | 0.467 |
| | 0.01 | 0.01 | 0.00 | 0.03 | 0.04 |
| Muscle | 0.085 | 0.036 | 0.010 | 0.005 | 0.004 |
| | 0.01 | 0.00 | 0.00 | 0.00 | 0.00 |
| Bone | 3.25 | 3.41 | 3.97 | 4.07 | 4.25 |
| | 0.11 | 0.39 | 0.41 | 0.18 | 0.05 |
| Kidney | 2.84 | 1.02 | 0.62 | 0.36 | 0.35 |
| | 0.95 | 0.09 | 0.21 | 0.02 | 0.06 |
| Bone/blood | 8 | 20 | 91 | 228 | 358 |
| | 0.7 | 3.4 | 5.2 | 1.1 | 13.5 |

| | | | | | |
|---|---|---|---|---|---|
| The lower row indicates standard deviation | | | | | |

**[Table 2]**

| 18F-Na | | | | | |
|---|---|---|---|---|---|
| | Time after administration | | | | |
| %I. D. /g | 15 min | 30 min | 60 min | 120 min | 240 min |
| Blood | 0.415 | 0.175 | 0.045 | 0.011 | 0.005 |
| | 0.01 | 0.03 | 0.01 | 0.00 | 0.00 |
| Liver | 0.358 | 0.159 | 0.036 | 0.007 | 0.003 |
| | 0.01 | 0.05 | 0.00 | 0.00 | 0.00 |
| Spleen | 0.276 | 0.145 | 0.039 | 0.006 | 0.002 |
| | 0.00 | 0.03 | 0.00 | 0.00 | 0.00 |
| Small intestine | 0.288 | 0.223 | 0.179 | 0.047 | 0.008 |
| | 0.02 | 0.02 | 0.01 | 0.01 | 0.00 |
| Large intestine | 0.209 | 0.136 | 0.159 | 0.305 | 0.300 |
| | 0.03 | 0.02 | 0.03 | 0.04 | 0.08 |
| Muscle | 0.134 | 0.069 | 0.022 | 0.004 | 0.001 |
| | 0.01 | 0.00 | 0.00 | 0.00 | 0.00 |
| Bone | 4.55 | 6.08 | 6.89 | 6.86 | 6.91 |
| | 0.29 | 0.36 | 0.39 | 0.32 | 0.24 |
| Kidney | 1.36 | 0.89 | 0.16 | 0.04 | 0.01 |
| | 0.09 | 0.49 | 0.01 | 0.02 | 0.00 |
| Bone/blood | 11 | 35 | 153 | 636 | 1491 |
| | 0.5 | 3.1 | 15.5 | 90.8 | 81.6 |

| | | | | | |
|---|---|---|---|---|---|
| The lower row indicates standard deviation | | | | | |

**[Table 3]**

| 68Ga-NOTA-alendronate | | | | | |
|---|---|---|---|---|---|
| | Time after administration | | | | |
| %I. D. /g | 15 min | 30 min | 60 min | 120 min | 240 min |
| Blood | 0.399 | 0.145 | 0.030 | 0.005 | 0.003 |
| | 0.01 | 0.01 | 0.00 | 0.00 | 0.00 |
| Liver | 0.081 | 0.039 | 0.020 | 0.015 | 0.016 |
| | 0.00 | 0.00 | 0.00 | 0.00 | 0.00 |
| Spleen | 0.103 | 0.046 | 0.021 | 0.014 | 0.012 |
| | 0.00 | 0.00 | 0.00 | 0.00 | 0.00 |
| Small intestine | 0.121 | 0.070 | 0.056 | 0.073 | 0.011 |
| | 0.01 | 0.01 | 0.01 | 0.04 | 0.00 |
| Large intestine | 0.132 | 0.054 | 0.023 | 0.019 | 0.077 |
| | 0.02 | 0.00 | 0.00 | 0.01 | 0.03 |
| Muscle | 0.085 | 0.033 | 0.008 | 0.004 | 0.002 |
| | 0.00 | 0.00 | 0.00 | 0.00 | 0.00 |
| Bone | 3.64 | 4.17 | 4.59 | 5.19 | 4.75 |
| | 0.19 | 0.37 | 0.59 | 0.18 | 0.14 |
| Kidney | 3.43 | 0.89 | 0.43 | 0.37 | 0.28 |
| | 2.09 | 0.16 | 0.11 | 0.01 | 0.06 |
| Bone/blood | 9 | 29 | 155 | 958 | 1696 |
| | 0.3 | 3.7 | 5.7 | 64.8 | 78.9 |

| | | | | | |
|---|---|---|---|---|---|
| The lower row indicates standard deviation | | | | | |

Compared to 99mTc-MDP, 68Ga-NOTA-alendronate shows more rapid clearance of radioactivity from blood but an equivalent value of accumulation in bone. As a result, a ratio of radioactivity accumulation in bone to that in blood is dominantly improved from an early stage after administration. Also, as shown in the Table, 68Ga-NOTA-alendronate exhibited a similar amount of non-specific accumulation in other organs to that of 99mTc-MDP, showing that there was almost no non-specific accumulation.

Further, 68Ga-NOTA-alendronate has exhibited a similar ratio of radioactivity accumulation in bone to that in blood to that of 18F-Na, and thus 68Ga-NOTA-alendronate is considered useful as a PET bone diagnostic agent.

### Example 6

### Rat imaging experiment

To Wistar male rats, 68Ga-NOTA-alendronate and 99m Tc-MDP were each administered into the rat tail vein in amounts of 18.5 to 37 MBq under anesthesia. Then, 60 and 180 minutes after administration, images were taken by PET/CT or a SPECT camera.

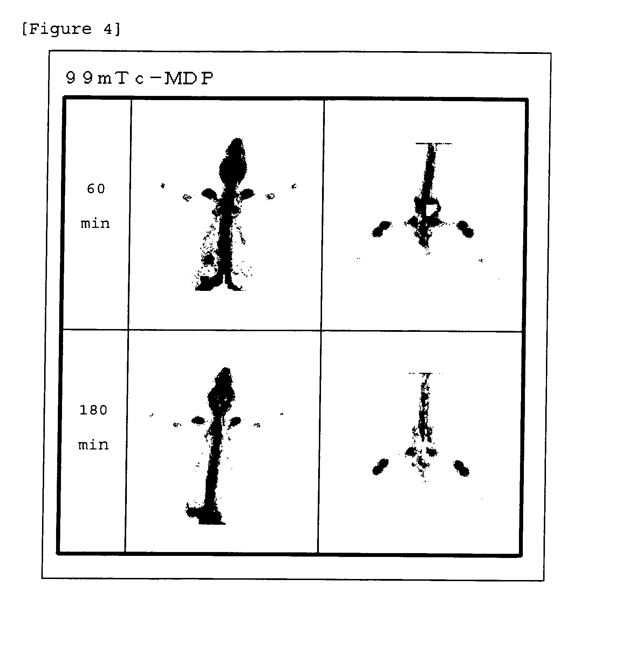

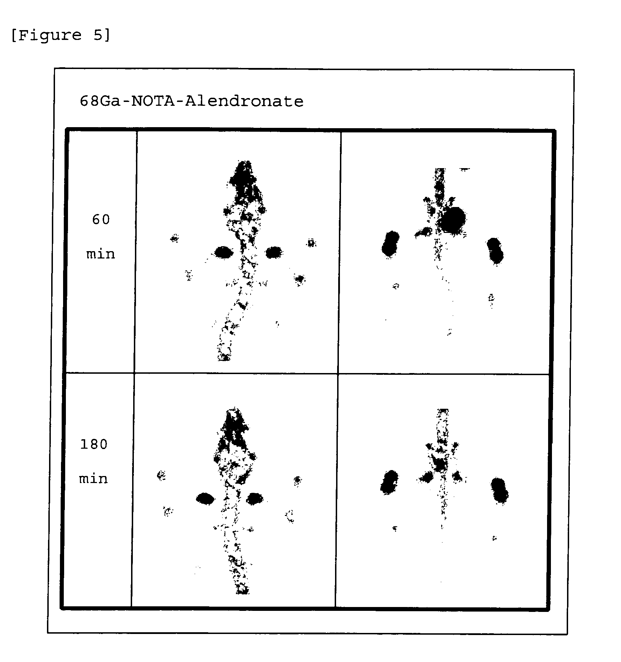

The results thus obtained are shown in Figures 4 and 5.

Owing to faster clearance of radioactivity of 68Ga-NOTA-alendronate from blood than that of 99mTc-MDP, bone is clearly visualized from 60 minutes after administration. From this, it is considered that 68Ga-NOTA-alendronate might enable diagnosis of bone lesion from an earlier stage after administration than 99mTc-MDP.

### Example 7

### Imaging experiment of local bone metastasis model mouse

Human prostate cancer-derived cells were transplanted into the right tibia of a mouse to produce a local bone metastasis model. To this model mouse, approximately 6.0 MBq of 68Ga-NOTA-alendronate was administered into the rat tail vein under anesthesia. An image was taken by PET/CT 60 minutes after administration.

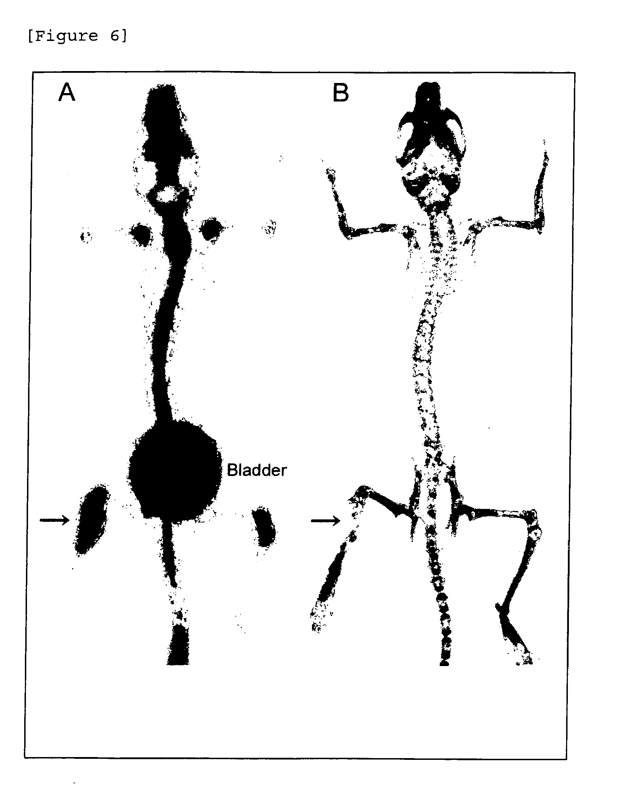

The results thus obtained are shown in Figure 6.

Because 68Ga-NOTA-alendronate shows high accumulation in bone and rapid clearance of radioactivity from blood, bone and the arrowed bone lesion are clearly visualized from 60 minutes after administration. From this, it is considered that 68Ga-NOTA-alendronate might enable diagnosis of bone lesion from an earlier stage after administration than existing preparations.

### Example 8

### Synthesis of DOTA-alendronate

DOTA-alendronate, in which alendronate is linked to 1,4,7,10,-tetraazacyclododecane-N,N',N",N"'-tetraacetic acid (DOTA), which is a cyclic chelate compound, via a spacer, was synthesized. Then, 68Ga-DOTA-alendronate, which is DOTA-alendronate labeled with 68Ga, was synthesized, to make a comparison of drug performance.

DOTA-alendronate was prepared by the steps 12 to 13 as shown below.

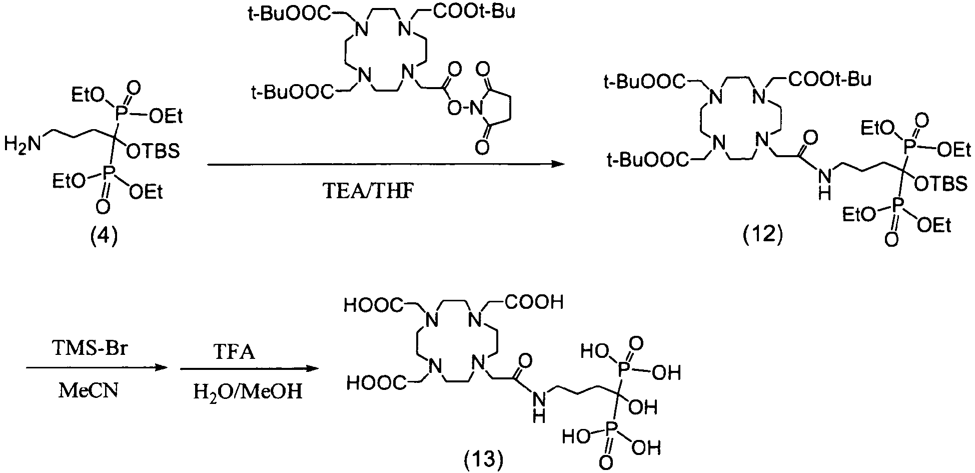

### Synthesis of tert-butyl-2-[4,10-di[2-(tert-butoxy)-2-oxoethyl] -7- (2-{ [4-{ [1- (tert-butyl) -1, 1-dimethylsilyl]oxy}-4,4-di(diethoxyphosphoryl)butyl]amino}-2-oxoethyl)-1,4,7,10-tetraazacyclododecanyl]acetate (12)

To tetrahydrofuran, 4-amino-1-{[(tert-butyl)-1,1-dimethylsilyl]oxy}-1,1-di(diethoxyphosphoryl)-butane (4) (100 mg) and tris-tert-butyl-DOTA-succinimide (100 mg) were dissolved, to which triethylamine was added, followed by stirring. The solvent was distilled off and the residue thus obtained was purified by silica gel column chromatography to give the title compound (120 mg). ESI-MS(posi)m/z:1030(M+H)⁺.

### Synthesis of 2-[4,10-di(carboxymethyl)-7-{2-[(4-hydroxy-4,4-diphosphonobutyl)amino]-2-oxoethyl}-1,4,7,10-tetraazacyclododecanyl)acetic acid ((13): DOTA-alendronate)

To tert-butyl-2- [4, 10-di [2- (tert-butoxy) -2-oxoethyl]-7-(2-{[4-{[1-(tert-butyl)-1,1-dimethylsilyl]oxy}-4,4-di(diethoxyphosphoryl)butyl]amino}-2-oxoethyl)-1,4,7,10-tetraazacyclododecanyl]acetate (12) (110 mg) dissolved in acetonitrile, bromotrimethylsilane (2 mL) was added, followed by stirring. Subsequently, water was added and the resulting solution was stirred, and the solvent was distilled off. To the residue thus obtained, water and acetonitrile were sequentially added, and the resulting precipitate was collected by filtration to give the title compound (70 mg) .
ESI-MS(nega)m/z:316(M-2H)²⁻,634 (M-H)⁻.

### Example 9

### Synthesis of 68Ga-DOTA-alendronate

Into a reaction container, 67 µL of sodium acetate buffer (pH 5.0) was added, to which 213 µL of a 68GaCl₃ solution was then added. Subsequently, 20 µL of a solution of DOTA-alendronate dissolved in sodium acetate buffer (pH 4.5) was added, and allowed to react at 100°C for 15 minutes. The resulting product was then left to stand at room temperature. The radiochemical purity was found to be 95% or higher as analyzed by HPLC and TLC.

### Example 10

### In vivo distribution of 68Ga-DOTA-alendronate in rats

To groups, each containing three Wistar male rats, 0.2 mL of each of 68Ga-DOTA-alendronate and 99mTc-MDP (product name: Techne MDP injection, manufactured by FUJIFILM RI Pharma Co., Ltd., 740 MBq/2 mL), each diluted to 12.3 to 19.8 MBq/mL, was administered into the rat tail vein under anesthesia. Then, 120 minutes after administration, the rats were sacrificed, organs were excised, and the weight and the radioactivity of each organ were measured. The measurement values were calculated as %I.D./g tissue weight, which is the radioactivity in each tissue divided by the tissue weight, as the ratio with respect to the dose.

The results of the measurement of the in vivo distribution of 68Ga-DOTA-alendronate in rats are shown in Table 4.

**[Table 4]**

| In vivo distribution 120 minutes after drug administration | | |
|---|---|---|
| %I. D. /g | 99mTc-MDP | 68Ga-DOTA-alendronate |
| Blood | 0.021 | 0.024 |
| | 0.00 | 0.00 |
| Liver | 0.024 | 0.018 |
| | 0.00 | 0.00 |
| Spleen | 0.020 | 0.020 |
| | 0.00 | 0.00 |
| Small intestine | 0.224 | 0.522 |
| | 0.17 | 0.27 |
| Large intestine | 0.015 | 0.055 |
| | 0.00 | 0.04 |
| Muscle | 0.007 | 0.005 |
| | 0.00 | 0.00 |
| Bone | 4.38 | 4.54 |
| | 0.10 | 0.42 |
| Kidney | 0.44 | 0.26 |
| | 0.05 | 0.04 |
| Bone/blood | 209 | 189 |

| | | |
|---|---|---|
| The lower row indicates standard deviation | | |

At the time of 120 minutes after administration, 68Ga-DOTA-alendronate exhibited high radioactivity accumulation in bone as 99mTc-MDP did. On the other hand, it exhibited delayed clearance from blood, resulting in no improvement in the ratio of radioactivity accumulation in bone/blood two hours after administration. It is considered that this delayed clearance from blood might be attributable to the stability of the 68Ga-DOTA complex in vivo.

Based on the foregoing, compared to 68Ga-DOTA-alendronate, 68Ga-NOTA-alendronate can clearly visualize bone at an early stage after administration, and it is considered that 68Ga-NOTA-alendronate enables diagnosis of a bone lesion from an earlier stage.

### Industrial Applicability

Compared to conventional radioactive bone diagnostic agents (for example, 99mTc-MDP), the radioactive bone diagnostic agent of the present invention shows equivalent accumulation in bone, while having faster clearance from blood. Thus, a high ratio of radioactivity accumulation in bone/blood is obtainable from an early stage after administration, enabling shortening of the time to imaging. Further, taking advantage of the characteristics of a PET nuclide, the radioactive bone diagnostic agent of the present invention allows easy tomographic imaging of the whole body, holding promise for improving the bone disease diagnostic capability.

## Claims

1. A bisphosphonic acid derivative represented by the chemical formula (II): 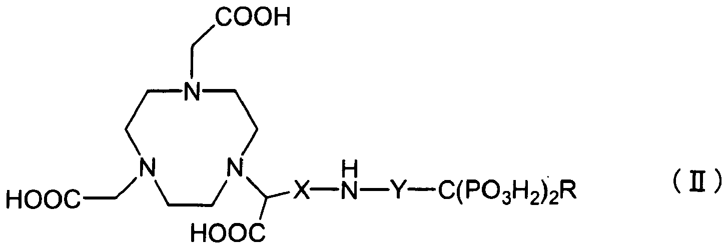 or a salt thereof, wherein X represents -(CH₂)ₘCO-, Y represents -(CH₂)ₙ-, R represents H, OH, or a halogen atom, m and n are independent of each other and m represents an integer of 1 to 3, and n represents an integer of 0 to 4.

2. A bisphosphonic acid derivative represented by the chemical formula (I): 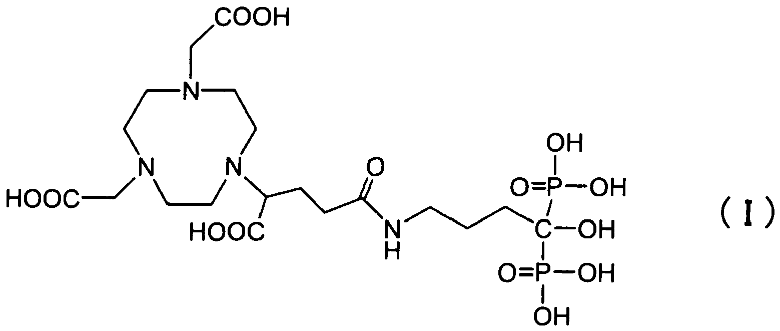 or a salt thereof.

3. A bisphosphonic acid derivative labeled with a radioactive metal nuclide or a salt thereof, wherein the radioactive metal nuclide coordinates to the bisphosphonic acid derivative or a salt thereof according to Claim 1 or 2.

4. The bisphosphonic acid derivative labeled with a radioactive metal nuclide or a salt thereof according to Claim 3, wherein the radioactive metal nuclide is selected from 62Cu, 64Cu, 67Cu, 67Ga, 68Ga, 177Lu, 153Sm, 90Y, and 111In.

5. A radioactive bone diagnostic agent comprising, as an active ingredient, the bisphosphonic acid derivative labeled with a radioactive metal nuclide or a salt thereof according to Claim 3, wherein the radioactive metal nuclide is selected from 62Cu, 64Cu, 67Ga, 68Ga, and 111In.

6. A radioactive bone therapeutic agent comprising, as an active ingredient, the bisphosphonic acid derivative labeled with a radioactive metal nuclide or a salt thereof according to Claim 3, wherein the radioactive metal nuclide is selected from 64Cu, 67Cu, 177Lu, 153Sm, and 90Y.

7. A kit for the preparation of a diagnostic or therapeutic radioactive agent, which comprises the bisphosphonic acid derivative or a salt thereof according to Claim 1 or 2 and is prepared by addition of a radioactive metal nuclide.

8. The bisphosphonic acid derivative labeled with a radioactive metal nuclide or a salt thereof according to Claim 3, for use in radioactive bone diagnosis, wherein the radioactive metal nuclide is selected from 62Cu, 64Cu, 67Ga, 68Ga, and 111In.

9. The bisphosphonic acid derivative labeled with a radioactive metal nuclide or a salt thereof according to Claim 3, for use in radioactive bone therapy, wherein the radioactive metal nuclide is selected from 64Cu, 67Cu, 177Lu, 153Sm, and 90Y.

10. A method of radioactive bone diagnosis, **characterized in that** the method comprises administrating an effective amount of the bisphosphonic acid derivative labeled with a radioactive metal nuclide or a salt thereof according to Claim 3, wherein the radioactive metal nuclide is selected from 62Cu, 64Cu, 67Ga, 68Ga, and 111In, to a subject in need thereof.

## Patentansprüche

1. Biphosphonsäurederivat der chemischen Formel (II): 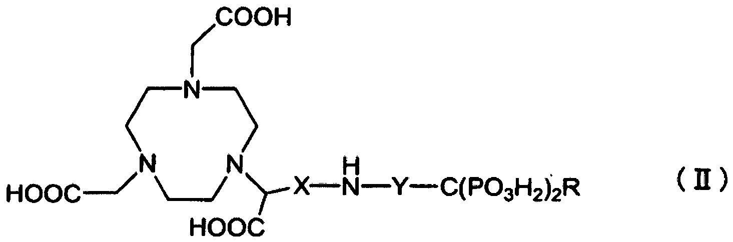 oder deren Salz, worin X für -(CH₂)ₘCO-, Y für -(CH₂)ₙ-, R für H, OH oder ein Halogenatom steht, m und n unabhängig voneinander sind und m für eine ganze Zahl von 1 bis 3 steht und n für eine ganze Zahl von 0 bis 4 steht.

2. Biphosphonsäurederivat der chemischen Formel (I): 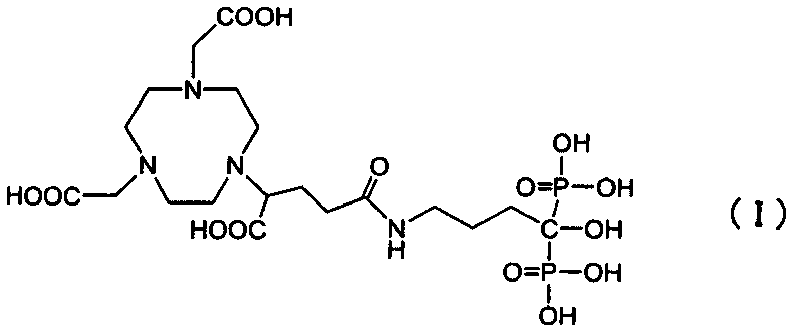 oder ein Salz davon.

3. Biphosphonsäurederivat, das mit einem radioaktivem Metallnuklid markiert ist, oder ein Salz davon, worin das radioaktive Metallnuklid an das Biphosphonsäurederivat oder ein Salz davon gemäß Anspruch 1 oder 2 koordiniert.

4. Das Biphosphonsäurederivat, das mit einem radioaktivem Metallnuklid markiert ist, oder ein Salz davon gemäß Anspruch 3, worin das radioaktive Metallnuklid ausgewählt ist aus 62Cu, 64Cu, 67Cu, 67Ga, 68Ga, 177Lu, 153Sm, 90Y und 111In.

5. Radioaktives Knochendiagnosemittel umfassend als aktiven Wirkstoff das Biphosphonsäurederivat, das mit einem radioaktivem Metallnuklid markiert ist, oder ein Salz davon gemäß Anspruch 3, worin das radioaktive Metallnuklid ausgewählt ist aus 62Cu, 64Cu, 67Ga, 68Ga und 111In.

6. Radioaktives Knochentherapiemittel umfassend als aktiven Wirkstoff das Biphosphonsäurederivat, das mit einem radioaktivem Metallnuklid markiert ist, oder ein Salz davon gemäß Anspruch 3, worin das radioaktive Metallnuklid ausgewählt ist aus 64Cu, 67Cu, 177Lu, 153Sm und 90Y.

7. Kit zur Herstellung eines diagnostischen oder therapeutischen radioaktiven Mittels, das das Biphosphonsäurederivat oder ein Salz davon gemäß Anspruch 1 oder 2 enthält und durch Zugabe eines radioaktiven Metallnuklids hergestellt wird.

8. Das Biphosphonsäurederivat, das mit einem radioaktiven Metallnuklid markiert ist, oder ein Salz davon gemäß Anspruch 3, zur Verwendung bei der radioaktiven Knochendiagnose, worin das radioaktive Metallnuklid ausgewählt ist aus 62Cu, 64Cu, 67Ga, 68Ga und 111In.

9. Das Biphosphonsäurederivat, das mit einem radioaktivem Metallnuklid markiert ist, oder ein Salz davon gemäß Anspruch 3, zur Verwendung in der radioaktiven Knochentherapie, worin das radioaktive Metallnuklid ausgewählt ist aus 64Cu, 67Cu, 177Lu, 153Sm und 90Y.

10. Verfahren zur radioaktiven Knochendiagnose, **dadurch gekennzeichnet, dass** das Verfahren eine Verabreichung einer wirksamen Menge des Biphosphonsäurederivats, das mit einem radioaktiven Metallnuklid markiert ist, oder eines Salzes davon gemäß Anspruch 3 an einen Patienten, der dies benötigt, umfasst, worin das radioaktive Metallnuklid ausgewählt ist aus 62Cu, 64Cu, 67Ga, 68Ga und 111In.

## Revendications

1. Dérivé d'acide bisphosphonique représenté par la formule générale (II) : 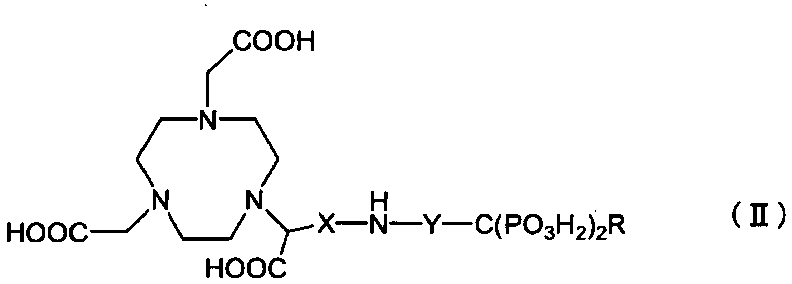 ou un sel de celui-ci, formule dans laquelle X représente -(CH₂)ₘCO-, Y représente - (CH₂)ₙ-, R représente H, OH ou un atome d'halogène, m et n sont indépendants l'un de l'autre et m représente un entier de 1 à 3 et n représente un entier de 0 à 4.

2. Dérivé d'acide bisphosphonique représenté par la formule chimique (I) : 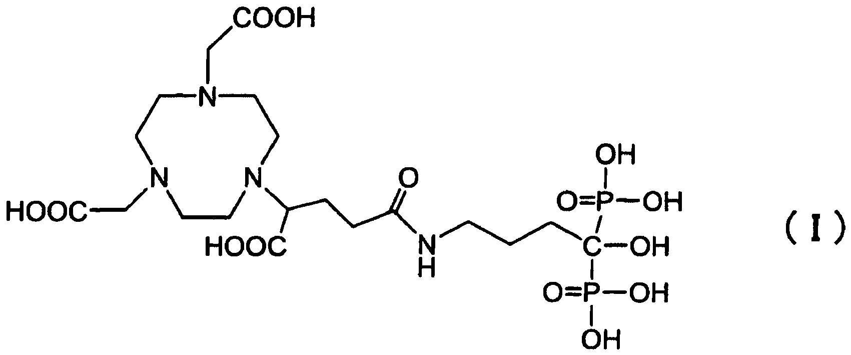 ou un sel de celui-ci.

3. Dérivé d'acide bisphosphonique marqué avec un nucléide métallique radioactif ou un sel de celui-ci, dans lequel le nucléide métallique radioactif se coordonne au dérivé d'acide bisphosphonique ou à un sel de celui-ci selon la revendication 1 ou 2.

4. Dérivé d'acide bisphosphonique marqué avec un nucléide métallique radioactif ou un sel de celui-ci selon la revendication 3, dans lequel le nucléide métallique radioactif est choisi parmi 62Cu, 64Cu, 67Cu, 67Ga, 68Ga, 177Lu, 153Sm, 90Y, et 111In.

5. Agent diagnostique osseux radioactif comprenant, en tant qu'ingrédient actif, le dérivé d'acide bisphosphonique marqué avec un nucléide métallique radioactif ou un sel de celui-ci selon la revendication 3, dans lequel le nucléide métallique radioactif est choisi parmi 62Cu, 64Cu, 67Ga, 68Ga, et 111In.

6. Agent thérapeutique osseux radioactif comprenant, en tant qu'ingrédient actif, le dérivé d'acide bisphosphonique marqué avec un nucléide métallique radioactif ou un sel de celui-ci selon la revendication 3, dans lequel le nucléide métallique radioactif est choisi parmi 64Cu, 67Cu, 177Lu, 153Sm, et 90Y.

7. Kit pour la préparation d'un agent radioactif diagnostique ou thérapeutique, qui comprend le dérivé d'acide bisphosphonique ou un sel de celui-ci selon la revendication 1 ou 2 et est préparé par addition d'un nucléide métallique radioactif.

8. Dérivé d'acide bisphosphonique marqué avec un nucléide métallique radioactif ou un sel de celui-ci selon la revendication 3, pour utilisation en diagnostic osseux radioactif, dans lequel le nucléide métallique radioactif est choisi parmi 62Cu, 64Cu, 67Ga, 68Ga, et 111In.

9. Dérivé d'acide bisphosphonique marqué avec un nucléide métallique radioactif ou un sel de celui-ci selon la revendication 3, pour utilisation en traitement osseux radioactif, dans lequel le nucléide métallique radioactif est choisi parmi 64Cu, 67Cu, 177Lu, 153Sm, et 90Y.

10. Méthode de diagnostic osseux radioactif, **caractérisée en ce qu'**elle comprend l'administration d'une quantité efficace du dérivé d'acide bisphosphonique marqué avec un nucléide métallique radioactif ou d'un sel de celui-ci selon la revendication 3, dans laquelle le nucléide métallique radioactif est choisi parmi 62Cu, 64Cu, 67Ga, 68Ga, et 111In, à un sujet en ayant besoin.
